# EUROPEAN PATENT SPECIFICATION

(11) **EP 0 967 210 B1**
(45) Date of publication and mention of the grant of the patent: **04.06.2003**
(21) Application number: 96941664.3
(22) Date of filing: 20.12.1996
(51) Int. Cl.: C07D 249/08, C07C 243/18, C07C 241/02

(54) **PROCESS FOR PREPARING BIOLOGICALLY ACTIVE DERIVATIVES OF 1,2,4-TRIAZOL AND INTERMEDIARIES USEFUL IN THIS PROCESS**
VERFAHREN ZUR HERSTELLUNG BIOLOGISCH AKTIVER DERIVATE DES 1,2,4-TRIAZOLS UND IN DIESEM VERFAHREN NUETZLICHE ZWISCHENPRODUKTE
PROCEDE DE PREPARATION DE DERIVES BIOLOGIQUEMENT ACTIFS DE 1,2,4-TRIAZOLE ET INTERMEDIAIRES UTILISES DANS CE PROCEDE

(30) Priority: 21.05.1996 SI 9600165
(43) Date of publication of application: 29.12.1999
(73) Proprietor: KRKA, tovarna zdravil, d.d., Novo mesto, 8501 Novo mesto (SI)
(72) Inventor: PALOMO COLL, Alberto, E-08017 Barcelona (ES)
(74) Representative: UEXKÜLL & STOLBERG
(86) International application number: ES9600245
(87) International publication number: WO97044330

## Description

### Technical Field

The invention belongs to the field of organic chemical synthesis and relates to a process for the preparation of a biologically active derivative of 1,2,4-triazole having the common name fluconazole, which is an important antifungal compound, from monosubstituted hydrazines and s-triazine. This antifungal compound is used in human medicine, veterinary medicine and agriculture.

### Technical Problem

There was a need for a simplified and industrially suitable process for the synthesis of fluconazole, which is a bistriazole antifungal active substance for human use (GB 2,099,818 (1982)), Merck Index, 11th Edition, p. 645.

### Prior Art

Synthesis of fluconazole, chemical name 2-[2,4-difluorophenyl]-1,3-bis[1H-1,2,4-triazole-1-yl)-propane-2-ol, of the formula (I) 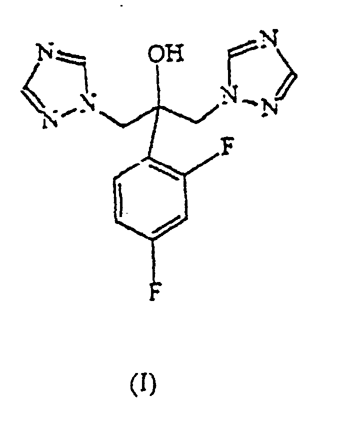 is disclosed in GB 2,099,818, US 4,404,216, YU 42770, ES 512,882 and ES 520,794, ES 618,198, WO 95/07895, AT 900,961.

In said patents two main synthesis routes are used, which are schematically disclosed in Schemes 1 and 2.

The synthesis routes of fluconazole are performed by opening the oxirane derivative (II) with 1,2,4-triazole (Scheme 1 below) or by the substitution of the reactive groups with 1,2,4-triazole on the intermediate (III), both times in a strongly alkaline medium. At the substitution of the reactive groups X (X represents halo, hydroxy, a substituted hydroxy group) in the reaction, an epoxide derivative is formed, which is, however, not isolated during the reaction (Scheme 2 below).

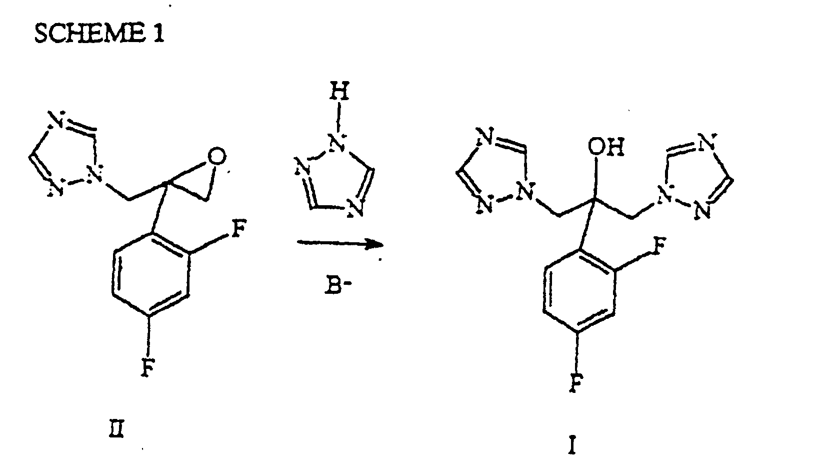

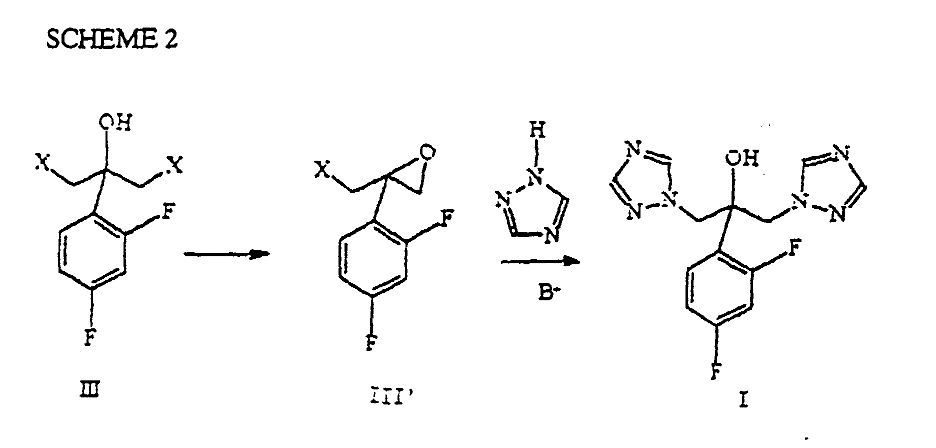

In both routes the reaction is carried out in an alkaline medium and 1,2,4-triazole represents a nucleophil. 1,2,4-triazole is partially reacted also in 4-position and thus undesired isomeric products are formed. Such a reactivity also results in a low reaction yield and, due to the presence of isomeric products, in a lesser purity of the product. When the reaction of 1,2,4-triazole is carried out with the intermediate (III) wherein X represents chloro, the yield amounts to 26%.

In WO 9604256 and WO 9604257 compounds having a similar structure as compounds IV according to the invention are disclosed.

### The Inventive Solution

By means of the process according to the invention as disclosed below, the formation of undesired products being formed with 1,2,4-triazole under alkaline reaction conditions is prevented and the reaction yield is close to a quantitative conversion. The reaction mechanism and conditions differ from the ones known in the literature and the formation of isomeric 1,2,4-triazole products is avoided. The purity of the products is greater.

The first object of the invention is a process for the preparation of 2-[2,4-difluorophenyl]-1,3-bis[1H-1,2.4-triazole-1-yl]-propane-2-ol (fluconazole) of the formula (I) 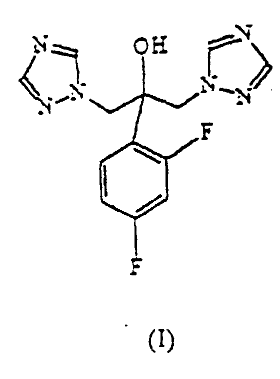 and of pharmaceutically acceptable salts thereof, characterized in that a compound of the formula (IV) 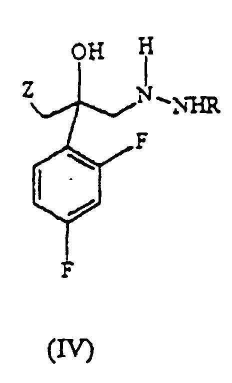 wherein R represents H, benzyl, triphenylmethyl and COOR¹ with R¹ being alkyl or aryl, preferably tert-butyl or ethyl, and Z represents a triazole radical or a hydrazine radical optionally substituted by R of the above definition, optionally in the salt form, is reacted with s-triazine of the formula 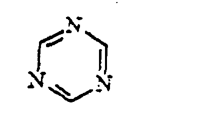 in an organic solvent under acidic reaction conditions.

As organic solvents there may be used alcohols, preferably ethanol, methanol, 1- and 2-propanol, ethers such as diethyl ether, dipropyl ether, diisopropyl ether, methyl tert-butyl ether, acetonitrile, DMSO, DMF, DMAA (dimethyl acetamide), N-methylpyrrolidone.

To the solvent an inorganic or organic acid is added such as formic acid, acetic acid, trifluoroacetic acid, methanesulfonic acid, malonic acid, maleic acid, malic acid, chloroacetic acid, dichloroacetic acid, propanoic acid or paratoluenesulfonic acid, hydrochloric acid, sulfuric acid and nitric acid, preferably formic acid or trifluoroacetic acid.

Also organic acid alone may be used as an organic solvent.

If a mixture of acids e.g. of organic acids is used, it may be operated at different ratios of these acids.

The reaction temperature is between -60°C and the boiling point of the reaction mixture used.

For the preparation of pharmaceutically acceptable salts of fluconazole, there can be used formic acid, acetic acid, trifluoroacetic acid, methanesulfonic acid, malonic acid, maleic acid, malic acid, paratoluenesulfonic acid and also hydrochloric acid, sulfuric acid and nitric acid.

The compound (I) (fluconazole) is obtained in a high yield (80-100%) depending upon the used acid or solvent, the reaction time, the reaction temperature and the pH of the reaction medium.

In the reaction between 2-(2,4-difluorophenyl)-1-(1H-1,2,4-triazol-1-yl)-3-hydrazino-propane-2-ol (IV) and s-triazine in absolute alcohol and under addition of HCl, the yield is about 80%. When acetic acid is used as the solvent, the reaction is almost quantitative (over 95%) already after 45 minutes. When formic acid is used, the conversion is quantitative already after 20 minutes at a temperature between 0 and 5°C.

The performed reactions show that the conversion of hydrazine compounds (IV) in the reaction with s-triazine to the compound (I) is better and quicker in strong acidic reaction conditions with the compound (IV) being present in the salt form such as hydrochloride, sulfate, acetate, formate, methanesulfonate and others. As mentioned before, the compound (IV) may be used in the form of a base or in the form of the above salts.

The advantage of the present process is obvious since in the reaction between the compound (IV) and s-triazine 2-[2,4-difluorophenyl]-1,3-bis [1H-1,2,4-triazole-1-yl]-propane-2-ol (I) (fluconazole) is formed quantitatively. The reaction is per-formed in acidic and strongly acidic reaction conditions (pH is 1-6). All processes known insofar for the synthesis of fluconazole have been carried out in basic reaction conditions (pH is 7-14).

The mechanism of the reaction of s-triazine with the compound (IV) wherein R is H and Z is 1,2,4-triazole radical is represented in the following scheme.

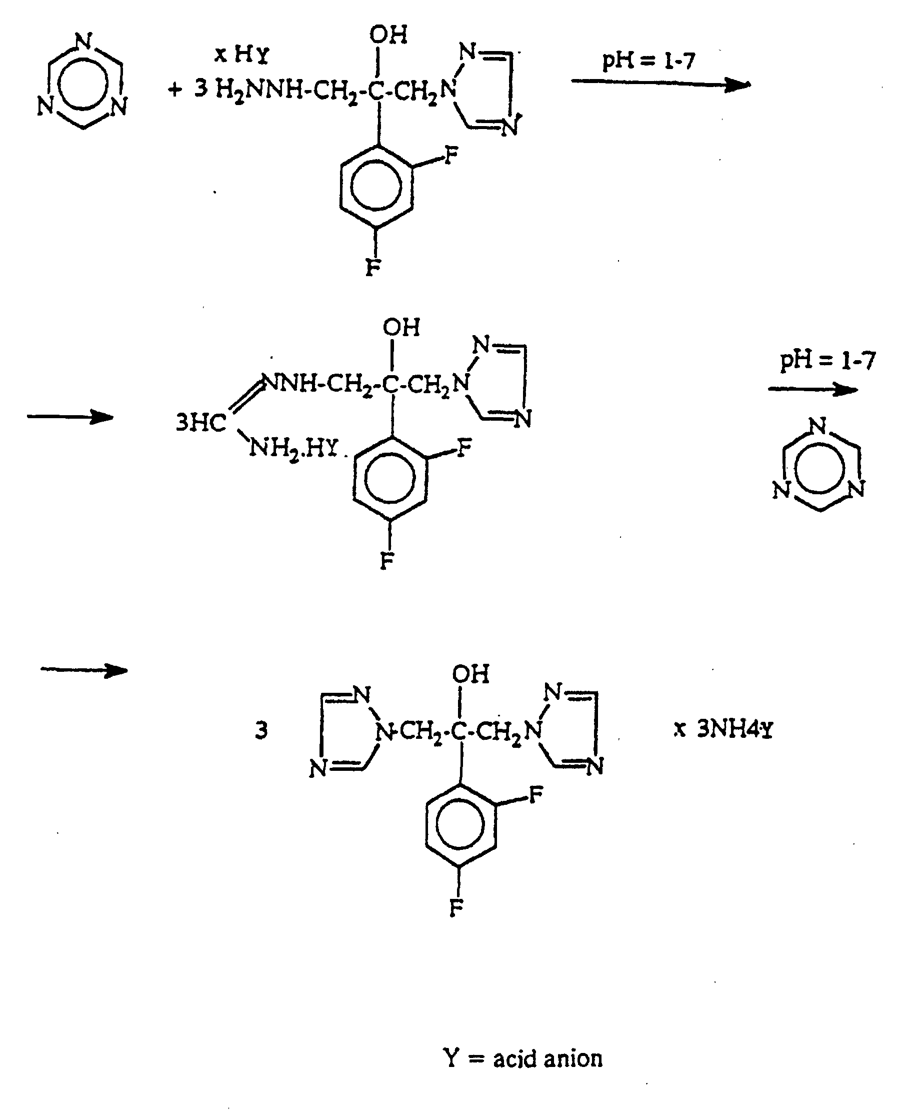

Compounds of the formula (IV) are novel and also represent an object of the invention. If they are used in salt form, these are salts with formic acid, acetic acid, trifluoroacetic acid, sulfonic acid, methanesulfonic acid, malonic acid, maleic acid, malic acid and paratoluenesulfonic acid as well as with hydrochloric acid, sulfuric acid, nitric acid.

The compound of the formula (IV) wherein Z represents a triazole radical is obtained according to one process variant as disclosed in Scheme 3. 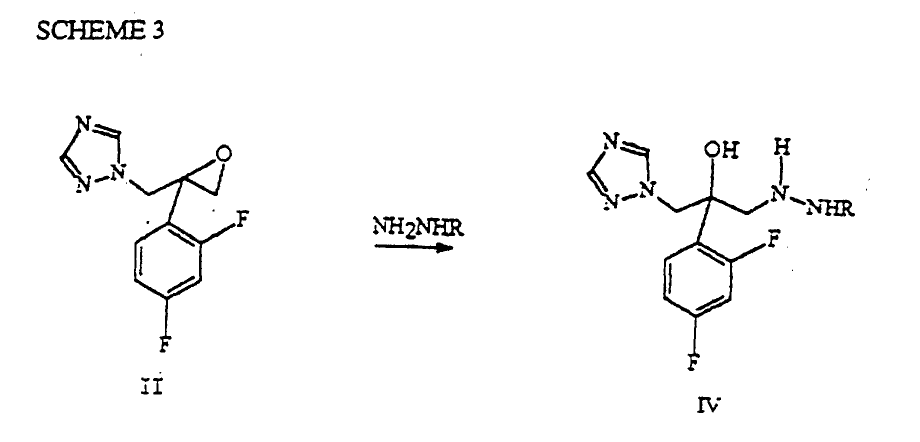 2-(2,4-difluorophenyl)-1-(1H-1,2,4-triazole-1-yl)propane-2,3-epoxide of the formula (II) is reacted with a hydrazine compound of the formula

NH₂NHR (V)

wherein R has the above meaning. The compound of the formula (V) may be used when R is hydrogen, in the form of hydrazine hydrate alone, hydrazine hydrate in water, hydrazine hydrate in organic solvents such as acetonitrile, or in a mixture of organic solvents and water, whereas in case that R has the remaining meanings different from hydrogen, it is used in an organic solvent

The compound of the formula (IV) wherein Z is a triazole rest is obtained according to another process variant as disclosed in Scheme 4.

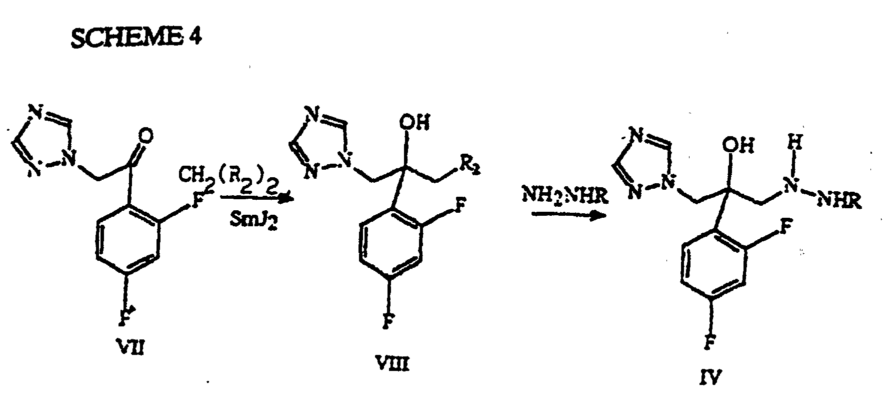

From 1-(2,4-difluorophenyl)-2-(1H-1,2,4-triazolo-1-yl)ethane-1-one (VII) by halo-methylating with dihalomethane CH₂(R₂)₂ wherein R₂ represents Cl, Br or I, and with a catalyst which may be samarium (II) iodide or samarium metal, 2-(2,4-difluorophenyl)-1-(1H-1,2,4-triazolo-1-yl)-3-halopropane-2-ol of the formula (VIII) wherein R₂ has the above meaning is obtained and then in a substitution reaction halo is exchanged for an appropriate hydrazine of the formula (V).

The compounds of the formula (IV) wherein Z is a hydrazine radical optionally substituted by R of the above meaning are obtained according to the process of the invention as disclosed in Scheme 5.

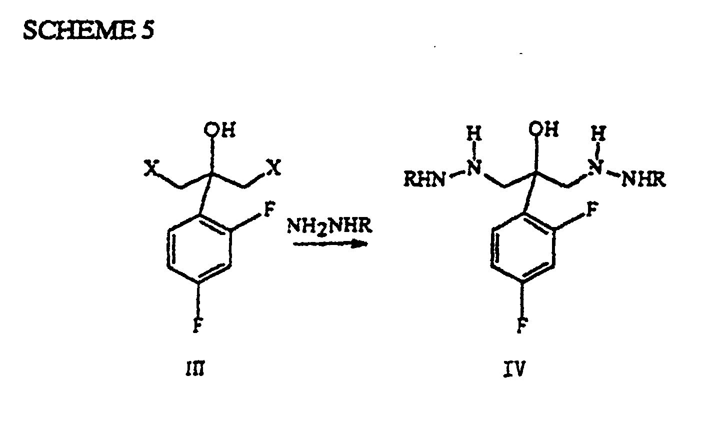

From 2-(2,4-difluorophenyl)-1,3-disubstituted propane-2-ol of the formula (III) wherein X represents halo, hydroxy or substituted hydroxy group, a compound of the formula (IV) is obtained by a substitution of the group X with a hydrazine compound of the formula V. If R in the compound of the formula (V) represents hydrogen the substitution may be performed with an excess of hydrazine hydrate or with hydrazin hydrate in a minimum excess in an organic solvent, preferably acetonitrile.

All these processes for the preparation of the compound of the formula (IV) are all carried out at a temperature between -25°C and the reflux of the reaction mixture. It is operated in an organic solvent.

An object of the invention is also a process for the preparation of the compound of the formula (IV) wherein Z is a hydrazine radical optionally substituted by R of the above meaning, characterized in that from 2-(2,4-difluorophenyl)-1,3-disubstituted propane-2-ol of the formula (III) 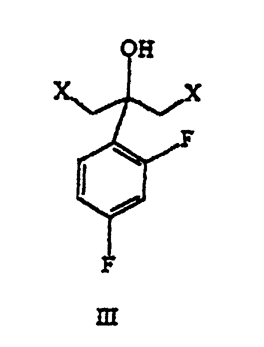 wherein X has the meaning of halo, hydroxy or a substituted hydroxy group, a compound of the formula (IV) is obtained by the substitution of the group X with the hydrazine compound of the formula (V).

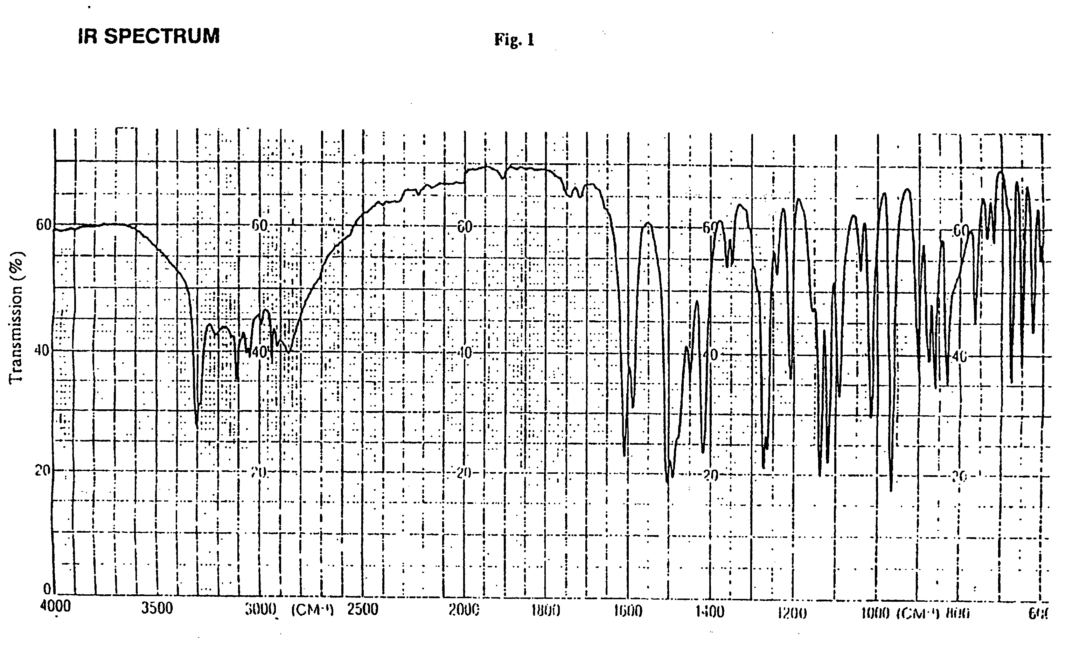

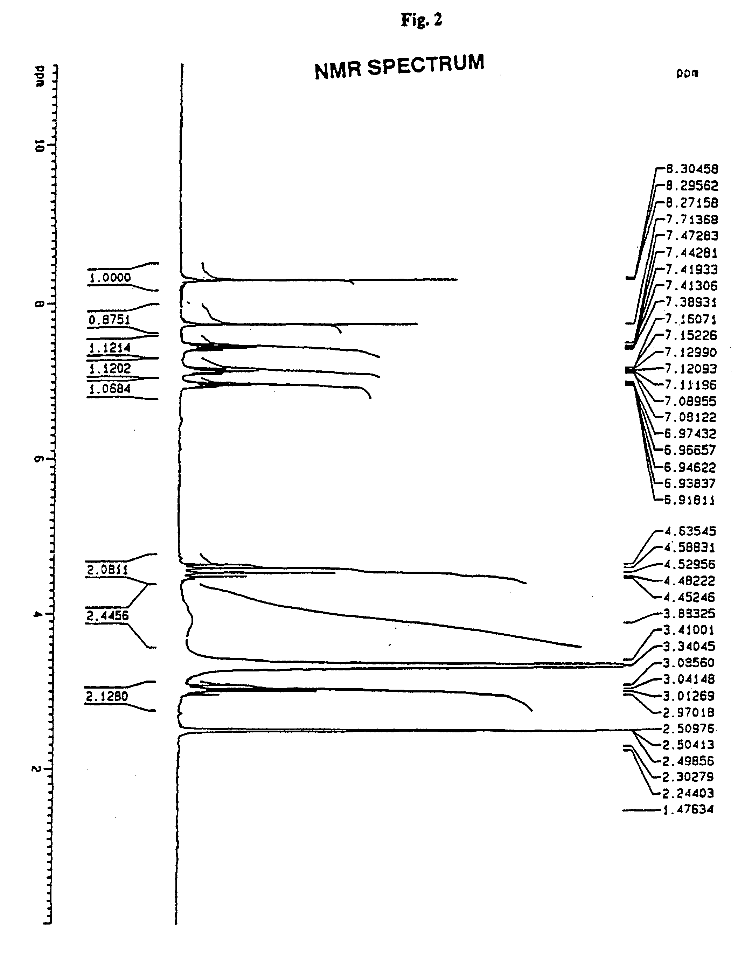

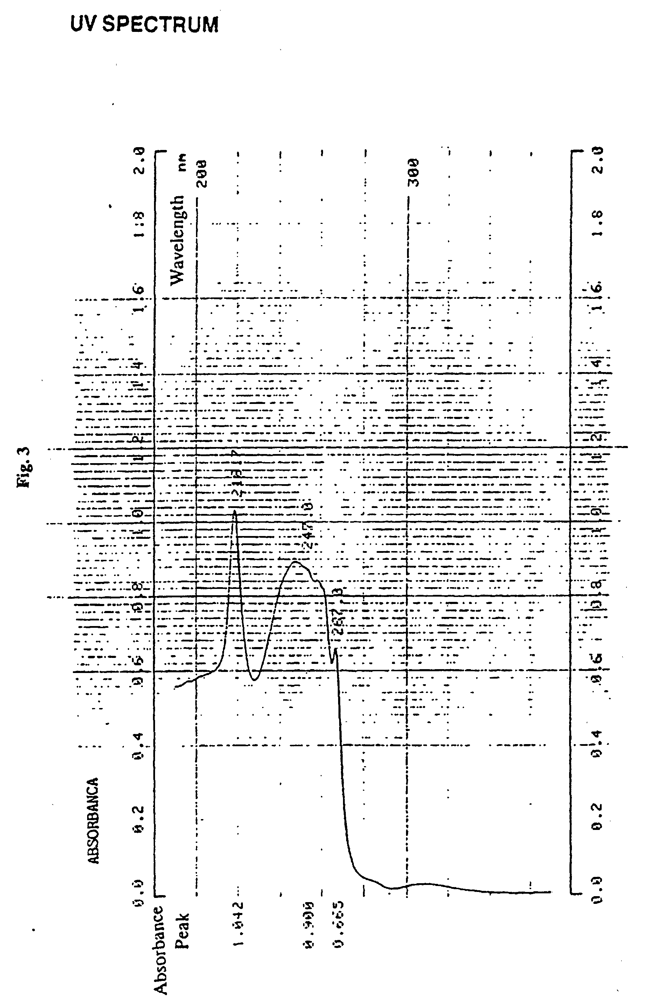

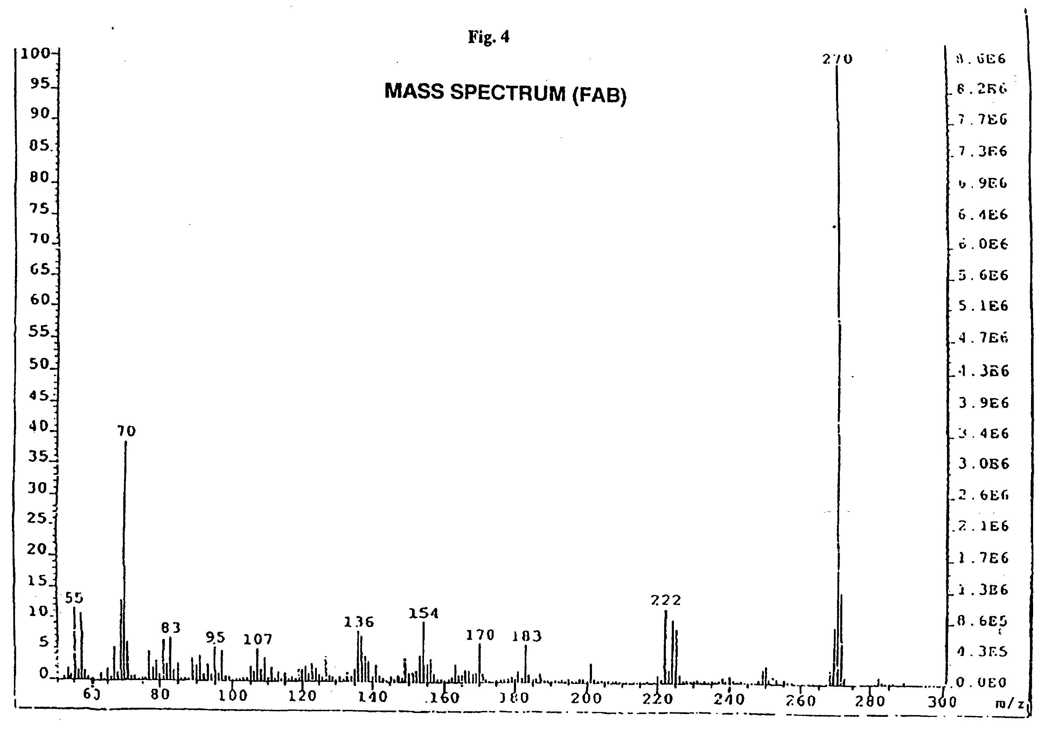

In Figs. 1, 2, 3 and 4 the IR spectrum, NMR spectrum, UV spectrum and mass spectrum (FAB) of the compound of the formula (IV) wherein R is hydrogen and Z is a 1,2,4-triazole radical are shown.

The invention is illustrated by the following Examples.

### Example 1

### Synthesis of hydrazine compound of the formula (IV) (Z is a triazole radical, R is H) from the compound of the formula (II)

To acetonitrile (12.5 ml) and epoxide (1.67 g, 0.005 mole) of the formula (II) in the form of methanesulfonate, hydrazine hydrate (0.61 ml, 0.0125 mole; 99%) was added. The reaction mixture was in three phases: two uncoloured phases and a suspension phase with a white solid. The reaction mixture was heated to the boiling point where only a two-phase system of two uncoloured phases was observed. The reaction mixture was heated at the boiling point for 3 hours when by TLC (chloroform/ methanol 15/2) the completion of reaction was established. The reaction mixture was cooled to 0-5°C in 10 minutes. The obtained white precipitate was filtered off and washed with acetonitrile. (Hydrazine methanesulfonate (0.64 g) was obtained.) The filtrate was evaporated at reduced pressure and on a bath having a temperature not over 40°C. A white product (1.35 g) (IV), 2-(2,4-difluorophenyl)-1-(1H-1,2,4-triazole-1-yl)-3-hydrazino-propane-2-ol, was obtained.

### Example 2

### Synthesis of hydrazine compound of the formula (IV) (Z is a triazole radical, R is H) from the compound of the formula (II)

It was proceeded as in Example 1. After the completed reaction the reaction mixture was given into a separating funnel, methylene chloride (25 ml) and a saturated aqueous NaCl solution (15 ml) were added. The mixture was shaken, the organic phase and the aqueous phase were separated, the aqueous phase was washed with methylene chloride (5 ml), the organic phases were combined, dried with anhydrous sodium sulfate, the solvent was evaporated in vacuo (the pressure was to be under 1.33 mbar) and the title compound (1.28 g) was obtained, which crystallized in the refrigerator.

### Example 3

### Synthesis of hydrazine compound of the formula (IV) (Z is a triazole radical, R is EtOOC)

A mixture of acetonitrile (10 ml), the epoxide of the formula (II) (0.333 g; 0.001 mole) and ethyl carbazate (0.520 g; 0.005 mole) was heated for 4 hours at reflux temperature. After the completed reaction the reaction mixture was cooled and chloroform (25 ml) and saturated aqueous NaCl solution (15 ml) were added. The mixture was shaken in a separating funnel, the organic phase was separated from the aqueous one and dried with anhydrous sodium sulfate. The solvent was evaporated and an oily product (0.314 g) was isolated.
IR (oily film): 3290, 3060, 3020, 2940, 1690, 1600, 1480, 1250,1130, 945, 835.
Mass spectrum (FAB) m/z: 342 (MH⁺)
¹H NMR (CDCl₃, 300 MHz) δ (ppm): 1.26 (t, 3H, ³J = 7.1 Hz), 2.88 (d, 1H, ²J = 4.7 Hz), 2.95 (d, 1H, ²J = 4.7 HZ), 3.76 (broad signal, 2H, -NHNH-), 4.16 (q, 2H, ³J = 7.1 Hz), 4.52 (d, 1H, ²J = 14.8 Hz), 4.82 (d, 1H, ²J = 14.8 Hz), 6.85 (m, 2H), 7.17 (m, 1H), 7,87 (s, 1H), 8.07 (s, 1H).

### Example 4

### Synthesis of hydrazine compound of the formula (IV) (Z is a triazole radical, R is-Bu^{t}OOC)

A mixture of acetonitrile (10 ml), the epoxide of the formula (II) (0.333 g; 0.001 mole) and tert-butyl carbazate (0.660 g; 0.005 mole) was heated for 4 hours at reflux temperature. After the completed reaction the reaction mixture was cooled and chloroform (25 ml) and saturated aqueous NaCl solution (15 ml) were added. The mixture was shaken in a separating funnel, the organic phase was separated from the aqueous one and dried with anhydrous sodium sulfate. The solvent was evaporated and an oily product (0.328 g) was isolated.
IR (oily film): 3380, 3070, 3020, 2940, 1680, 1590, 1575, 1470, 1400, 1250, 1115, 945, 850.
Mass spectrum (FAB) m/z: 370 (MH⁺, 23%), 314 (100%), 289 (15%), 230 (24%), 177 (16%), 89 (12%), 70 (52%), 57 (40%).
¹HNMR (CDCl₃, 300 MHz) δ (ppm): 1.43 (s, 9H), 2.88 (d, 1H, ²J = 4.7 Hz), 2.95 (d, 1H, ²J = 4.7 Hz), 4.51 (d, 1H, ²J = 14.8 Hz), 4.83 (d, 1H ²J = 14.8 Hz), 6.84 (m, 2H), 7.17 (m, 1H), 7.86 (s, 1H), 8.07 (s, 1H).

### Example 5

### Synthesis of 2-(2,4-difluorophenyl)-1-(1H-1,2,4-triazolo-1-yl)-3-iodopropane-2-ol of the formula (VIII)

Into redistilled dry THF (10 ml) diiodomethane (2 mmole; 536 mg) was given under argon and 0.1 M THF solution (25 ml) of samarium (II) iodide was added. Then gradually a THF solution of 1-(2,4-difluorophenyl)-2-(1H-1,2,4-triazolo-1-yl)ethane-1-one (VII) (223 mg; 1 mmol) was added. After the addition the solution was stirred for another 30 minutes at room temperature. The obtained precipitate was filtered by suction, the solvent was evaporated in vacuo, methylene chloride (20 ml) was added to the residue, the solution was dried with sodium sulfate, the solvent was evaporated and a crude product was crystallized from isopropanol. The title compound (335 mg; 82%) was obtained.
IR: 3120, 2970, 2960, 1610, 1500, 1420, 1275, 1140, 1105, 970, 855, 680
¹H NMR (CDCl₃, 300 MHz) δ (ppm): 2.88 (d, 1H, ²J = 4.7 Hz), 2.95 (d, 1H, ²J = 4.7), 3.58 (d, 1H, ²J = 10.7 Hz), 3.76 (d, 1H, ²J = 10.7 Hz), 6.8 (m, 2H), 7.2 (m, 1H), 7.87 (s, 1H), 8.07 (s, 1H).

### Example 6

### Synthesis of hydrazine compound of the formula (IV) (Z is a triazole radical, R is H) from the compound of the formula (VIII)

The compound of the formula (VIII) (365 mg; 1 mmole) obtained in Example 5, hydrazine hydrate (125 mg; 2.5 mmole) and acetonitrile (10 ml) were heated for 3 hours at reflux temperature. After the completed reaction dichloromethane (15 ml) and saturated aqueous NaCl solution (10 ml) were added, the mixture was shaken in a separating funnel, the organic phase was separated from the aqueous one, dried with anhydrous sodium sulfate, the solvent was evaporated in vacuo and the title compound (241 mg; 89.6%) was obtained.

### Example 7

### Synthesis of fluconazole

To a crude hydrazine compound (IV) from Example 1 (1.35 g; 0.005 mole) triazine (1.42 g; 0.017 mole; 97%) and acetic acid (10 ml) were added. A yellowish reaction mixture was heated at reflux temperature (116-118°C) for 2 hours and 45 minutes. During this period the yellow reaction mixture became orange. The completion of the reaction was detected by TLC (chloroform/methanol 15/2). Acetic acid was evaporated at reduced pressure and at the bath temperature of 70-75°C. Water (10 ml) and NaCl (3 g) were added to the orange residue after evaporation and methylene chloride (25 ml) was added. The pH of the aqueous phase was 2-3. The pH of the aqueous phase was adjusted to 7 with a 33% NaOH solution and then it was decanted. The aqueous phase was washed with methylene chloride (10 ml), the organic phases were combined and dried with anhydrous sodium sulfate. It was filtered, washed with methylene chloride and methylene chloride was evaporated at the temperature of 40°C and at reduced pressure. A pink solid (1.49 g; 97.4%) was isolated.
TLC: chloroform/metanol 15/2, Rf 0.51.

The spectroscopic data (IR, ¹H NMR, mass spectrum) corresponded to the title compound.

### Example 8

### Synthesis of fluconazole

To the hydrazine compound (IV) (1.35 g; 0.005 mole) from Example 1 triazine (1.42 g; 0.017 mole; 97%) and formic acid (10 ml; 98%) were added. The obtained yellowish reaction mixture was heated at the reflux temperature (100-102°C) for 40 minutes when the reaction mixture became orange, and the completion of the reaction was detected by TLC. Formic acid was evaporated at reduced pressure and at the bath temperature of 70-71°C. Water (10 ml) and NaCl (3 g) and methylene chloride (25 ml) were added to the residue. The pH of the aqueous phase was 2-3. The pH of the solution was adjusted to 7 with a 33% NaOH solution and it was decanted. The organic phase was washed with methylene chloride (10 ml), the organic phases were combined and dried with anhydrous sodium sulfate. It was filtered, washed with methylene chloride and methylene chloride was evaporated at the temperature of 40°C and at reduced pressure. A lightly coloured product (1.48 g; 96,7%) was isolated.
TLC: chloroform/metanol 15/2, Rf 0.51.

The spectroscopic data (IR, ¹H NMR, mass spectrum) corresponded to the title compound.

### Example 9

### Synthesis of fluconazole

It was proceeded as in Example 8. The reaction time was 70 minutes at reflux temperature (100-101°C). A slightly coloured product (1.44 g; 94.1%) was obtained.
TLC: chloroform/metanol 15/2, Rf 0.51.

The spectroscopic data (IR, ¹H NMR, mass spectrum) corresponded to the title compound.

### Example 10

### Synthesis of fluconazole

It was proceeded as in Example 8. The reaction time was 50 minutes at 80-85°C A slightly coloured product (1.50 g; 98%) was obtained.
TLC: chloroform/metanol 15/2, Rf 0.51.

The spectroscopic data (IR, ¹H NMR, mass spectrum) corresponded to the title compound.

### Example 11

### Synthesis of fluconazole

To the hydrazine compound (IV) (1.35 g; 0.005 mole) from Example 1, triazine (1.42 g; 0.017 mole; 97%) and formic acid (10 ml; 98%) were added. The yellowish reaction mixture was heated to the temperature of 30°C and then heated for 75 minutes at 30-35°C. The yellow reaction mixture became orange and the completion of the reaction was detected by TLC (chloroform/methanol 15/2). Water (10 ml) and NaCl (3 g) and methylene chloride (25 ml) were added to the reaction mixture. The pH of the aqueous phase was 1.15. The pH of the solution was adjusted to 7 with a 33% NaOH solution and an uncoloured aqueous phase and a yellowish organic phase were decanted. The phases were separated, the aqueous phase was washed with methylene chloride (10 ml), organic phases were combined and dried with anhydrous sodium sulfate. It was filtered, the drying agent was washed with methylene chloride and the solvent was evaporated at the temperature of 40°C and at reduced pressure. A white product (1.49 g; 97.4%) was isolated.
TLC: chloroform/metanol 15/2, Rf 0.51.

The spectroscopic data (IR, ¹H NMR, mass spectrum) corresponded to the title compound.

### Example 12

### Synthesis of fluconazole

It was proceeded as in Example 11. The reaction time was 2 hours 35 minutes at the temperature 20-25°C. A slightly coloured product (1.51 g; 98.7%) was obtained.
TLC: chloroform/metanol 15/2, Rf 0.51.

The spectroscopic data (IR, ¹H NMR, mass spectrum) corresponded to the title compound.

### Example 13

### Synthesis of fluconazole

It was proceeded as in Example 11. The reaction time was 1 hour at the temperature 0-5°C. A slightly coloured product (1.50 g; 98%) was obtained.
TLC: chloroform/metanol 15/2, Rf 0.51

The spectroscopic data (IR, ¹H NMR, mass spectrum) corresponded to the title compound.

### Example 14

### Synthesis of fluconazole

It was proceeded as in Example 11. The reaction time was 90 minutes at the temperature 0-5°C. A white product (1.46 g; 95%) was obtained.
TLC: chloroform/metanol 15/2, Rf 0.51

The spectroscopic data (IR, ¹H NMR, mass spectrum) corresponded to the title compound.

### Example 15

### Synthesis of fluconazole

It was proceeded as in Example 11. The reaction time was 1 hour at the temperature 0-5°C. 97% triazine (0.7 g; 0.009 mole) was used. A slightly coloured product (1.46 g; 95.4%) was obtained.
TLC: chloroform/metanol 15/2, Rf 0.51

The spectroscopic data (IR, ¹H NMR, mass spectrum) corresponded to the title compound.

### Example 16

### Synthesis of fluconazole

To a hydrazine compound (IV) (1.35 g; 0.005 mole) from Example 1 methylene chloride (20 ml), trifluoroacetic acid (2 ml; 0.026 mole) and s-triazine (0.8 g; 0.00987 mole) were added. A two-phase system was heated to 37°C and formic acid (4 ml; 98%) was added to obtain a yellow solution. The reaction mixture was kept for 1 hour at 35°C. TLC showed that the reaction was completed. Then a solution of water (10 ml) and NaCl (3 g) was added. The pH was 0.28. A 33% NaOH solution was added to pH 7.56 and then it was decanted. The aqueous phase was colourless and the organic phase was slightly pink. The aqueous phase was washed with methylene chloride (10 ml), organic phases were combined, dried with anhydrous sodium sulfate, filtered, washed with methylene chloride and evaporated on rotavapor in vacuo (bath temperature 40°C). A white product (1.39 g; yield 91%) was obtained.
TLC (chloroform/methanol 15/2) showed that the product was very pure (one stain); Rf 0.51.

The spectroscopic data (IR, ¹H NMR, mass spectrum) corresponded to the title compound.

### Example 17

### Synthesis of fluconazole

To the hydrazine compound (IV) (1.35 g; 0.005 mole) from Example 1, methylene chloride (10 ml), formic acid (4 ml; 98%), trifluoroacetic acid (1.7 ml; 0.022 mole) and s-triazine (0.6 g; 0.0074 mole) were added. An orange red solution was obtained. The reaction mixture was kept for 1 hour at 25°C. TLC showed that the reaction was completed. Then a solution of water (10 ml) and NaCl (3 g) and methylene chloride (10 ml) were added. The pH was 0.05. A 33% NaOH solution was added to pH 8.14 and then it was decanted. The aqueous phase was colourless and the organic phase was yellow. The aqueous phase was washed with methylene chloride (10 ml) and then again with methylene chloride (2 x 5 ml). Organic phases were combined, dried with anhydrous sodium sulfate, filtered, washed with methylene chloride and evaporated on rotavapor in vacuo (bath temperature 40°C). A beige product (1.48 g; yield 96.7%) was obtained.

The spectroscopic data (IR, ¹H NMR, mass spectrum) corresponded to the title compound.

### Example 18

### Synthesis of fluconazole

To the hydrazine compound (IV) (1.35 g; 0.005 mole) from Example 1, methylene chloride (40 ml), formic acid (4 ml; 98%), trifluoroacetic acid (1.7 ml; 0.022 mole) and s-triazine (0.6 g; 0.0074 mole) were added. An orange emulsion (two phases) was obtained, which means that the reaction took place in heterogenous phase. The reaction mixture was heated at reflux (41°C) and there were still two phases. Such reaction mixture was kept at reflux for 1 hour. TLC showed that the reaction was completed. Then the reaction mixture was cooled to room temperature and a solution of water (10 ml) and NaCl (3 g) was added. The pH was 0.17. A 33% NaOH solution was added to pH 8.45 and then it was decanted. The aqueous phase was colourless and the organic phase was almost colourless. The aqueous phase was washed with methylene chloride (10 ml) and then again with methylene chloride (2 x 5 ml). Organic phases were combined, dried with anhydrous sodium sulfate, filtered, washed with methylene chloride and evaporated on rotavapor in vacuo (bath temperature 40°C). A white solid (1.48 g; 96.7%) was obtained.
TLC (chloroform/methanol 15/2) showed that the product was very pure (one stain).

The spectroscopic data (IR, ¹H NMR, mass spectrum) corresponded to the title compound.

### Example 19

### Synthesis of fluconazole

To the hydrazine compound (IV) (1.35 g; 0.005 mole) from Example 1 methylene chloride (20 ml), formic acid (4 ml; 98%), trifluoroacetic acid (1.7 ml; 0.022 mole) and s-triazine (0.6 g; 0.0074 mole) were added. An orange solution (30°C) was obtained. The reaction mixture was kept at 30°C for 1 hour. TLC showed that the reaction was completed. A solution of water (10 ml) and NaCl (3 g) was added. The pH was 0.27. A 33% NaOH solution was added to pH 8 and then it was decanted. The aqueous phase was colourless and the organic phase was yellow. The aqueous phase was washed with methylene chloride (10 ml) and then again with methylene chloride (2 x 5 ml). Organic phases were combined, dried with anhydrous sodium sulfate, filtered, washed with methylene chloride and evaporated on rotavapor in vacuo (bath temperature abot 40°C). A beige solid (1.51 g; yield 98.7%) was obtained.

The spectroscopic data (IR, ¹H NMR, mass spectrum) corresponded to the title compound.

### Example 20

### Synthesis of fluconazole

To the hydrazine compound (IV) (1.35 g; 0.005 mole) from Example 1, methylene chloride (40 ml), formic acid (4 ml; 98%) and trifluoroacetic acid (1.7 ml; 0.022 mole) were added and the reaction mixture was cooled to -5°C. Then s-triazine (0.6 g; 0.0074 mole) was added and the reaction mixture was kept at -5°C to -10°C for 1 hour. Two phases were present, which means that the reaction took place in heterogenous phase. TLC showed that the reaction was completed. Then the temperature of the reaction mixture was increased to 15-20°C and a solution of water (10 ml) and NaCl (3 g) was added. The pH was 0.01. A 33% NaOH solution was added to pH 8.15 and then it was decanted. The aqueous phase was colourless and the organic phase was pale yellow. The aqueous phase was washed with methylene chloride (10 ml) and then again with methylene chloride (2 x S ml). Organic phases were then combined, dried with anhydrous sodium sulfate, filtered, washed with methylene chloride and evaporated on rotavapor in vacuo (bath temperature 40°C). A solid (1.49 g; yield 97.4%) was obtained.
TLC (chloroform/methanol 15/2) showed that the product was very pure (one stain).

The spectroscopic data (IR, ¹H NMR, mass spectrum) corresponded to the title compound.

### Example 21

### Synthesis of fluconazole

To the hydrazine compound (IV) (1.35 g; 0.005 mole) from Example 1, acetonitrile (20 ml), formic acid (4 ml; 98%) and trifluoroacetic acid (1.7 ml; 0.022 mole) were added and the reaction mixture was cooled to -5°C. Then s-triazine (0.6 g; 0.0074 mole) was added and a yellow solution was obtained. The system was kept at -5°C to -10°C for 1 hour and then the temperature was allowed to increase to 15-20°C and methylene chloride (20 ml) and a solution of water (10 ml) and NaCl (3 g) were added. The pH was 1.22. A 33% NaOH solution was added to pH 8.35 and then it was decanted. The aqueous phase was colourless and the organic phase was yellow. The aqueous phase was washed with methylene chloride (10 ml) and then again with methylene chloride (2 x 5 ml). Organic phases were then combined, dried with anhydrous sodium sulfate, filtered, washed with methylene chloride and evaporated on rotavapor in vacuo (bath temperature 40°C). A beige product (1.53 g; yield 100%) was obtained.
TLC (chloroform/methanol 15/2) showed that the product was pure (one stain).

### Example 22

### Synthesis of fluconazole

To the hydrazine compound (IV) (1.35 g; 0.005 mole) from Example 1, methylene chloride (40 ml), formic acid (4 ml; 98%) and trifluoroacetic acid (1.7 ml; 0.022 mole) were added and the reaction mixture was cooled to -5°C. Two phases were obtained. Then s-triazine (0.8 g; 0.00987 mole) was added. A yellow emulsion (two phases) was obtained, which means that the reaction took place in heterogenous phase. The reaction mixture was kept at -5°C to -10°C for 1 hour, then the temperature was allowed to increase to 15-20°C and a solution of water (10 ml) and NaCl (3 g) was added. The pH was 0.57. A 33% NaOH solution was added to pH 8.29 and then it was decanted. The aqueous phase was colourless and the organic phase was almost colourless. The aqueous phase was washed with methylene chloride (10 ml) and then again with methylene chloride (2 x 5 ml). Organic phases were then combined, dried with anhydrous sodium sulfate, filtered, washed with methylene chloride and evaporated on rotavapor in vacuo (bath temperature 40°C). A beige product (1.48 g; yield 96.7%) was obtained.
TLC (chloroform/methanol 15/2) showed that the product was very pure (one stain).

### Example 23

### Synthesis of fluconazole

To acetonitrile (12.5 ml) and epoxide (1.67 g, 0.005 mole) of the formula (II) in the form of methanesulfonate, hydrazine hydrate (0.61 ml, 0.0125 mole; 99%) was added. The reaction mixture was in three phases: two uncoloured phases and a suspension phase with a white solid. The reaction mixture was heated to the boiling point where only a two-phase system of two uncoloured phases was observed. The reaction mixture was heated at the boiling point for 3 hours when by TLC (chloroform/ methanol 15/2) the completion of reaction was established.

The reaction mixture was cooled to room temperature and then methanesulfonic acid (0.2 ml) was added, the hydrazine methansulfonate formed was filtered and then washed with acetonitrile (30 ml). Acetonitrile was evaporated on rotavapor and to the crude product 98% HCOOH (5 ml) and methylene chloride (5 ml) were added. The system was cooled to 0 to -5°C and s-triazine (0.6 g; made according to the procedure disclosed in W. Kantlehner et al., Synthesis, 1979, 690) was added to obtain a yellow orange solution. The system was kept for 1 hour at 0 to -5°C and afterwards a solution of water (10 ml) and NaCl (3 g) and also methylene chloride (15 ml) were added. A 33% NaOH solution was added to achieve pH 8.12 (approximately 12 ml of 33% NaOH) and decanted. The aqueous phase was washed once with 10 ml of methylene chloride, then with 5 ml of methylene chloride and again with 5 ml of methylene chloride. The organic phase was dried with anhydrous sodium sulfate, filtered, washed and evaporated on rotavapor for 2 hours with a bath temperature of 80°C. Yield 1.41 g (92%)

### Example 24

### Crystallization of fluconazole

### Variant I:

Crude fluconazole (2.50 g) was blended with isopropanol (20 ml) and a suspension was obtained. The reaction medium was heated to the temperature of 55°C when undissolved particles dissolved and a pink coloured solution was obtained. Active coal (0.5 g) was added and the reaction mixture was heated for 20 minutes at 55-65°C. Solid particles were filtered off and washed with isopropanol. The filtrate was concentrated under reduced pressure to a volume of 5 ml and cooled in 12 hours to 0-5°C. The obtained product was filtered and washed with isopropanol cooled to -11°C. A snow white precipitate (1.89 g) was obtained. Yield 80%.
M.p. of recrystallized product: 135-137°C.

The spectroscopic data (IR, ¹⁹F NMR, UV, mass spectrum) corresponded to the title compound.

The mother liquid and isopropanol used for washing were used for further recrystallizations:

To fluconazole (2.22 g) a solvent (20 ml: 7 ml of the mother liquid from the previous recrystallization and 13 ml of isopropanol) was added and the mixture was heated to 55°C. Active coal (0.5 g) was added and the reaction mixture was stirred for 20 minutes at 55-60°C, filtered at 55°C, washed with isopropanol and concentrated to 5 ml of isopropanol. It was cooled to -5°C in 12 hours and fluconazole was filtered off. It was washed with cooled isopropanole (3 x 2.5 ml). A snow white product (1.90 g) was isolated. Yield 90%.
M.p. of the recrystallized product: 135-138°C.

The spectroscopic data (IR, ¹⁹F NMR, UV, mass spectrum) corresponded to the title compound.

To the mother liquid water (30 ml) and methylene chloride (15 ml) were added. The aqueous phase was was decanted and extracted with methylene chloride (2 x 10 ml). Organic phases were combined and dried with anhydrous sodium sulfate, filtered and the solvent was evaporated under reduced pressure. An orange product (0.64 g) was isolated. Isopropanol (0.5 ml) was added and it was cooled to -5°C. The product obtained was filtered by suction.

### Variant II:

Crude fluconazole (2.5 g) was blended with ethyl acetate (20 ml) and heated to the boiling point, active coal (0.3 g) was added and the heating was continued under reflux for another 10 minutes. It was filtered and the filtrate was concentrated to a volume of 5 ml. It was cooled to 0°C and the obtained product was filtered by suction. A white product (2.25 g; 90%) with a m.p. 136-138°C was obtained. The mother liquid could be used for further recrystallizations.

### Example 25

### Synthesis of fluconazole methanesulfonate

Fluconazole (1 g; 0.00327 mole) was blended with methyl tert-butylether (10 ml) and a white suspension was obtained. Methanesulfonic acid (0.21 ml; 0.00327 mole) was added to the reaction mixture drop by drop. The reaction mixture was stirred at room temperature for 1 hour, filtered and the solid precipitate was washed with methyl tert-butylether (2 x 5 ml). A white precipitate (1.30 g) was isolated.
M.p.: 77-80°C.

The spectroscopic data (IR, ¹H NMR, UV, mass spectrum) corresponded to the title compound.

## Claims

1. A process for the preparation of 2-[2,4-difluorophenyl]-1,3-bis[1H-1,2,4-triazole-1-yl]-propane-2-ol (fluconazole) of the formula (I) 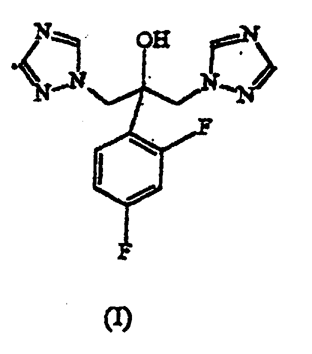 and of pharmaceutically acceptable salts thereof, **characterized in that** a compound of the formula (IV) 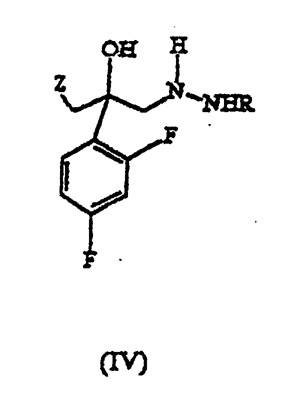 wherein R represents H, benzyl, triphenylmethyl and COOR¹ with R¹ being alkyl or aryl, and Z represents a triazole radical or a hydrazine radical optionally substituted by R of the above definition, optionally in the salt form, is reacted with s-triazine of the formula 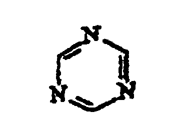 in an organic solvent under acidic reaction conditions as the reaction medium and at a temperature from -60°C to the reflux temperature of the reaction mixture.

2. A process according to claim 1 **characterized in that** R¹ is tert-butyl or ethyl.

3. A process according to claim 1 or 2 **characterized in that** as the reaction medium there are used
a) an organic solvent such as alcohols, ethers such as diethyl ether, dipropyl ether, diisopropyl ether, methyl tert-butyl ether, acetonitrile, DMSO, DMF, DMAA (dimethyl acetamide), N-methylpyrrolidone, and mineral and organic acids such as formic acid, acetic acid, trifluoroacetic acid, methanesulfonic acid, malonic acid, maleic acid, malic acid, chloroacetic acid, dichloroacetic acid, propanoic acid or paratoluenesulfonic acid, hydrochloric acid, sulfuric acid and nitric acid, or
b) an organic acid alone.

4. A process according to any one of claims 1 to 3, wherein the alcohol is ethanol, methanol, 1- or 2-propanol, and the organic acid is formic acid or trifluoroacetic acid.

5. Hydrazine compounds of the formula (IV) 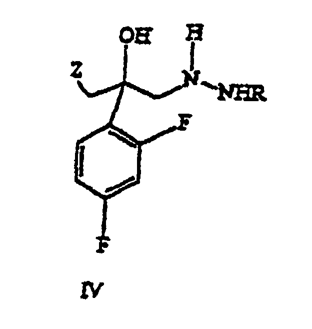 wherein R represents H, benzyl, triphenylmethyl and COOR¹ with R¹ being alkyl or aryl, and Z represents a triazole radical or hydrazine radical optionally substituted by R defined as above, with the exception of the compound wherein R is H and Z is a triazole radical, optionally in the salt form.

6. Hydrazine compounds according to claim 5, wherein R¹ is tert-butyl or ethyl.

7. Process for the preparation of the compound of the formula (IV) as defined in claim 5 or 6 wherein Z is a hydrazine radical optionally substituted by R of the above meaning, **characterized in that** from 2-(2,4-difluorophenyl)-1,3-disubstituted propane-2-ol of the formula (III) 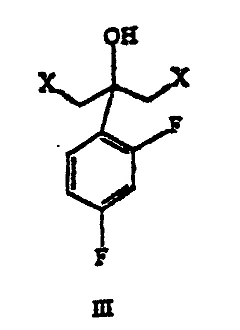 wherein X has the meaning of halo, hydroxy or a substituted hydroxy group, a compound of the formula (IV) is obtained by the substitution of the group X with a hydrazine compound of the formula (V)
NH₂NHR (V).

## Patentansprüche

1. Verfahren zur Herstellung von 2-[2,4-Difluorphenyl)-1,3-bis [1H-1,2,4-triazol-1-yl]-propan-2-ol (Fluconazol) der Formel (I) 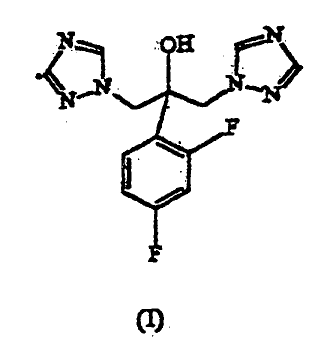 und von pharmazeutisch akzeptablen Salzen davon, **dadurch gekennzeichnet, dass** eine Verbindung der Formel (IV) 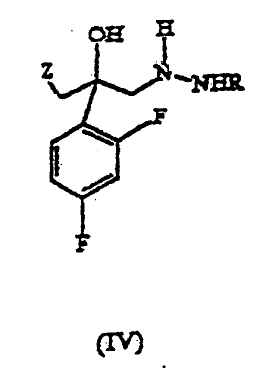 in der R H, Benzyl, Triphenylmethyl und COOR¹ repräsentiert, wobei R¹ Alkyl oder Aryl ist, und Z ein Triazolrest oder ein Hydrazinrest, gegebenenfalls substituiert durch R der obigen Definition, gegebenenfalls in der Salzform, repräsentiert, mit s-Triazin der Formel 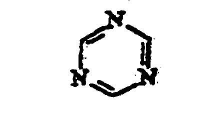 in einem organischen Lösungsmittel unter sauren Reaktionsbedingungen als dem Reaktionsmedium und bei einer Temperatur von -60 °C bis zur Rückflusstemperatur der Reaktionsmischung zur Reaktion gebracht wird.

2. Verfahren nach Anspruch 1, **dadurch gekennzeichnet, dass** R¹ tert.-Butyl oder Ethyl ist.

3. Verfahren nach Anspruch 1 oder 2, **dadurch gekennzeichnet, dass** als Reaktionsmedium
a) ein organisches Lösungsmittel, wie z.B. Alkohole, Ether, wie z.B. Diethylether, Dipropylether, Diisopropylether, Methyl-tert.-butylether, Acetonitril, DMSO, DMF, DMAA (Dimethylacetamid), N-Methylpyrrolidon und mineralische und organische Säuren, wie z.B. Ameisensäure, Essigsäure, Trifluoressigsäure, Methansulfonsäure, Malonsäure, Maleinsäure, Äpfelsäure, Chloressigsäure, Dichloressigsäure, Propansäure oder Para-Toluolsulfonsäure, Salzsäure, Schwefelsäure und Salpetersäure, oder
b) eine organische Säure allein.
verwendet wird.

4. Verfahren gemäß einem der Ansprüche 1 bis 3, bei dem der Alkohol Ethanol, Methanol, 1- oder 2-Propanol ist, und die organische Säure Ameisensäure oder Trifluoressigsäure ist.

5. Hydrazinverbindungen der Formel (IV) 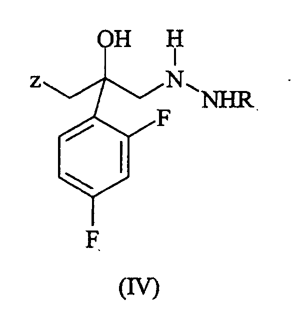 in der R H, Benzyl, Triphenylmethyl und COOR¹ repräsentiert, wobei R¹ Alkyl oder Aryl ist, und Z ein Triazolrest oder Hydrazinrest, gegebenenfalls substituiert durch R der obigen Definition, repräsentiert, gegebenenfalls in der Salzform, mit der Ausnahme der Verbindung, in der R H und Z ein Triazolrest ist.

6. Hydrazinverbindungen nach Anspruch 5, bei denen R¹ tert.-Butyl oder Ethyl ist.

7. Verfahren zur Herstellung einer Verbindung der Formel (IV), wie in Anspruch 5 oder 6, definiert, wobei Z ein Hydrazinrest, gegebenenfalls substituiert durch R der oben genannten Bedeutung, ist, **dadurch gekennzeichnet, dass** aus 2-(2,4-Difluorphenyl)-1,3-disubstituiertem Propan-2-ol der Formel (III) 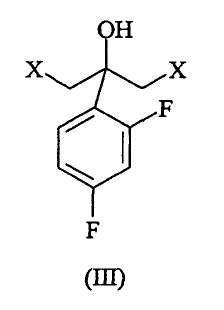 wobei X die Bedeutung von Halogen, Hydroxy oder einer substituierten Hydroxygruppe hat, eine Verbindung der Formel (IV) durch Substitution der Gruppe X mit einer Hydrazinverbindung der Formel (V),
NH₂NHR (V),
erhalten wird.

## Revendications

1. Procédé pour la préparation du 2-[2,4-difluorophényl]-1,3-bis[1H-1,2,4-triazol-1-yl]-propan-2-ol (fluconazole) de formule I 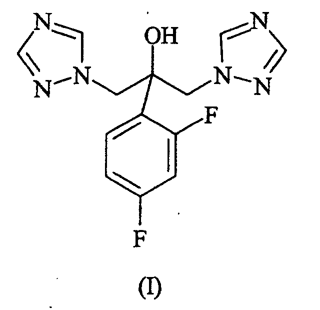 et des sels pharmaceutiquement acceptables de celui-ci, **caractérisé en ce qu'**un composé de formule ( IV) 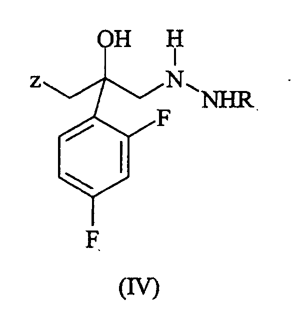 dans lequel R représente un H, un benzyle, un triphénylméthyle, et un COOR¹, R¹ étant un alkyle ou un aryle, et Z représente un radical triazole ou un radical hydrazine facultativement substitué par R défini comme ci-dessus, facultativement sous forme de sel, est mis à réagir avec de la s-triazine de formule 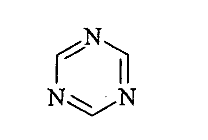 dans un solvant organique dans des conditions réactionnelles acides comme milieu réactionnel et à une température allant de -60 °C à la température de reflux du mélange réactionnel.

2. Procédé selon la revendication 1 **caractérisé en ce que** R¹ est un tert-butyle ou un éthyle.

3. Procédé selon la revendication 1 ou la revendication 2, **caractérisé en ce que** comme milieu réactionnel sont utilisés
a) un solvant organique tel que des alcools, des éthers tels que de l'éther diéthylique, de l'éther dipropylique, de l'éther diisopropylique, de l'éther tert-butylméthylique, de l'acétonitrile, du DMSO, du DMF, du DMAA (diméthylacétamide), de la N-méthylpyrrolidone, et des acides minéraux et organiques tels que de l'acide formique, de l'acide acétique, de l'acide trifluoroacétique, de l'acide méthanesulfonique, de l'acide malonique, de l'acide maléique, de l'acide malique, de l'acide chloroacétique, de l'acide dichloroacétique, de l'acide propionique ou de l'acide p-toluènesulfonique, de l'acide chlorhydrique, de l'acide sulfurique et de l'acide nitrique, ou
b) un acide organique seul.

4. Procédé selon l'une quelconque des revendications 1 à 3, dans lequel l'alcool est de l'éthanol, du méthanol, du 1-propanol ou du 2-propanol, et l'acide organique est de l'acide formique ou de l'acide trifluoroacétique.

5. Composés hydrazine de formule (IV) 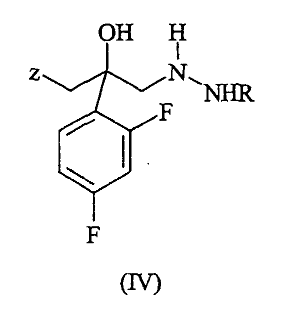 dans lequels R représente un H, un benzyle, un triphénylméthyle, et un COOR¹ avec R¹ étant un alkyle ou un aryle, et Z représente un radical triazole ou un radical hydrazine facultativement substitué par R défini comme ci-dessus, à l'exception du composé dans lequel R est un H et Z est un radical triazole, facultativement sous forme de sel.

6. Composés hydrazine selon la revendication 5, dans lesquels R¹ est un tert-butyle ou un éthyle.

7. Procédé de préparation du composé de formule (IV) selon la revendication 5 ou la revendication 6, dans lequel Z est un radical hydrazine facultativement substitué par R selon le sens ci-dessus, **caractérisé en ce que** à partir du propan-2-ol 2-(2,4-difluorophényl)-1,3-disubstitué de formule (III) 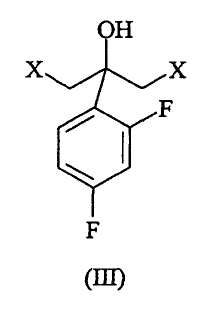 dans lequel X représente un groupe halo, hydroxy, ou hydroxy substitué, un composé de formule (IV) est obtenu par la substitution du groupe X par un composé hydrazine de formule (V)
NH₂NHR (V)
